(19) Europäisches Patentamt European Patent Office Office européen des brevets

(11) **EP 4 745 979 A1**

(12) **EUROPEAN PATENT APPLICATION**
published in accordance with Art. 153(4) EPC

(43) Date of publication:
20.05.2026 Bulletin 2026/21

(21) Application number: 24864301.7

(22) Date of filing: 30.07.2024

(51) International Patent Classification (IPC):
*G16H 20/30* (2018.01)

(52) Cooperative Patent Classification (CPC):
G16H 20/30

(86) International application number:
PCT/CN2024/108534

(87) International publication number:
WO 2025/055578 (20.03.2025 Gazette 2025/12)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 11.09.2023 CN 202311168990

(71) Applicant: Huawei Technologies Co., Ltd.
Shenzhen, Guangdong 518129 (CN)

(72) Inventors:
• ZHENG, Wenhui
  Shenzhen, Guangdong 518129 (CN)
• JIANG, Jin
  Shenzhen, Guangdong 518129 (CN)
• XIE, Wenyang
  Shenzhen, Guangdong 518129 (CN)
• XIE, Rujia
  Shenzhen, Guangdong 518129 (CN)

(74) Representative: Pfenning, Meinig & Partner mbB
Patent- und Rechtsanwälte
Theresienhöhe 11a
80339 München (DE)

(54) **HEALTH PLAN FORMULATION METHOD, HEALTH PLAN FORMULATION APPARATUS, AND ELECTRONIC DEVICE**

(57) This application provides a health plan formulation method, a health plan formulation apparatus, and an electronic device, and may be applied to the field of electronic devices. In the technical solutions provided in this application, the electronic device obtains a target weight loss rate proportion of a user and a current weight of the user, where the target weight loss rate proportion is a weight loss rate proportion expected by the user, and the weight loss rate proportion is a proportion of a weight loss in first unit duration in the current weight; determines weight loss fat content corresponding to the target weight loss rate proportion, where the weight loss fat content is a proportion of a fat amount in a weight loss in the weight loss; and determines a calorie deficit of the user in second unit duration based on the weight loss fat content corresponding to the target weight loss rate proportion, the target weight loss rate proportion, the current weight of the user, fat calories per unit weight, and lean body calories per unit weight. In this way, a matched fat loss workout plan is provided for the user.

FIG. 3

## Description

This application claims priority to Chinese Patent Application No. 202311168990.5, filed with the China National Intellectual Property Administration on September 11, 2023 and entitled "HEALTH PLAN FORMULATION METHOD, HEALTH PLAN FORMULATION APPARATUS, AND ELECTRONIC DEVICE", which is incorporated herein by reference in its entirety.

## TECHNICAL FIELD

Embodiments of this application relate to the field of electronic devices, and more specifically, to a health plan formulation method, a health plan formulation apparatus, and an electronic device.

## BACKGROUND

Electronic devices may obtain related information about users, and formulate health plans for the users based on the related information for reference by the users, to improve physical fitness of the users.

However, the health plans formulated by the electronic devices currently lack sufficient precision, which affects rates at which the physical fitness of the users is improved.

## SUMMARY

Embodiments of this application provide a health plan formulation method, a health plan formulation apparatus, and an electronic device, to provide a precise health plan for a user.

According to a first aspect, a health plan formulation method is provided. The method is applied to an electronic device and includes: obtaining a target weight loss rate proportion of a user and a current weight of the user, where the target weight loss rate proportion is a weight loss rate proportion expected by the user, and the weight loss rate proportion is a proportion of a weight loss in first unit duration in the current weight; determining weight loss fat content corresponding to the target weight loss rate proportion, where the weight loss fat content is a proportion of a fat amount in a weight loss in the weight loss; and determining a calorie deficit of the user in second unit duration based on the weight loss fat content corresponding to the target weight loss rate proportion, the target weight loss rate proportion, the current weight of the user, fat calories per unit weight, and lean body calories per unit weight.

In this method, the corresponding weight loss fat content is determined based on the target weight loss rate proportion of the user, and the calorie deficit is determined based on the weight loss fat content. Therefore, a more accurate calorie deficit can be obtained. This can help improve accuracy of a health fat loss plan formulated by the user based on the calorie deficit.

In some possible implementations, obtaining the target weight loss rate proportion of the user includes: obtaining a target weight loss rate of the user and the current weight of the user, where the target weight loss rate is a weight loss rate expected by the user, and the weight loss rate is a weight loss in the first unit duration; and determining the target weight loss rate proportion based on the target weight loss rate of the user and the current weight of the user.

In some possible implementations, determining the weight loss fat content corresponding to the target weight loss rate proportion includes: determining a group category to which the user belongs; determining a first mapping relationship from a plurality of mapping relationships based on the group category to which the user belongs, where the first mapping relationship is a mapping relationship corresponding to the group category to which the user belongs in the plurality of mapping relationships, the plurality of mapping relationships are in a one-to-one correspondence with a plurality of group categories, and each mapping relationship in the plurality of mapping relationships indicates a mapping relationship between a fat proportion in a weight loss of a user of a corresponding group category and a weight loss proportion of the user of the corresponding group category in the first unit duration; and determining, based on the first mapping relationship, the weight loss fat content corresponding to the target weight loss rate proportion.

In some possible implementations, determining the group category to which the user belongs includes: obtaining user information, where the user information includes at least one piece of the following information of the user: a maximum oxygen uptake, a body fat percentage, an age, a gender, the current weight, and a body mass index; and determining, based on the user information, the group category to which the user belongs.

In some possible implementations, the weight loss fat content corresponding to the target weight loss rate proportion, the target weight loss rate proportion, the current weight of the user, the fat calories per unit weight, the lean body calories per unit weight, and the calorie deficit of the user in the second unit duration satisfy the following relation:

$$g=(w*v*p*k1+w*v*(1-p)*k2)/t.$$

g represents the calorie deficit, w represents the current weight of the user, v represents the target weight loss rate proportion, p represents the weight loss fat content corresponding to the target weight loss rate proportion, k1 represents the fat calories per unit weight, k2 represents the lean body calories per unit weight, and t represents a ratio of the first unit duration to the second unit duration.

In some possible implementations, the method includes determining a diet plan and a workout plan of the user in the second unit duration based on the calorie deficit and basal metabolism calories of the user in the second unit duration.

According to a second aspect, an electronic device or a cloud server is provided. A method includes: obtaining a first basal metabolism condition, first fat loss duration, and a first workout capability, where the first basal metabolism condition is a basal metabolism condition of a user for whom a health plan is to be formulated, the first workout capability is a workout capability of the user for whom the health plan is to be formulated, and the first fat loss duration is fat loss duration expected by the user for whom the health plan is to be formulated; and determining a first target workout plan based on the first basal metabolism condition, the first fat loss duration, and the first workout capability, where the first target workout plan includes a plurality of target phases, a plurality of pieces of target duration, a plurality of target workout item sets, and a plurality of workout duration ratios, the plurality of pieces of target duration are in a one-to-one correspondence with the plurality of target phases, each piece of target duration in the plurality of pieces of target duration is duration included in the first fat loss duration for a corresponding target phase, the plurality of target workout item sets are in a one-to-one correspondence with the plurality of target phases, each target workout item set in the plurality of target workout item sets includes a workout item in the corresponding target phase, the plurality of workout duration ratios are in a one-to-one correspondence with the plurality of target workout item sets, and each workout duration ratio in the plurality of workout duration ratios is a workout duration ratio between workout items in a corresponding target workout item set.

In the method, a personalized fat loss workout plan is specified based on basic information, the workout capability, and a fat loss target of the user, and the personalized fat loss workout plan flexibly and accurately adapts to weight loss training requirements in various scenarios.

In some possible implementations, determining the first target workout plan based on the first basal metabolism condition, the first fat loss duration, and the first workout capability includes: determining the plurality of target phases, the plurality of pieces of target duration, a plurality of target workout type sets, and the plurality of workout duration ratios based on the first basal metabolism condition and the first fat loss duration, where the plurality of target workout type sets are in a one-to-one correspondence with the plurality of target phases, and each target workout type set in the plurality of target workout type sets includes a workout type in the corresponding target phase; and determining a target workout item set of the corresponding target phase based on the first workout capability and each target workout type set.

In some possible implementations, determining the plurality of target phases, the plurality of pieces of target duration, the plurality of target workout type sets, and the plurality of workout duration ratios based on the first basal metabolism condition and the first fat loss duration includes: determining the plurality of target phases and the plurality of pieces of target duration based on the first basal metabolism condition and the first fat loss duration; determining a target workout type set of each target phase in the plurality of target phases, to obtain a plurality of target workout type sets that are in a one-to-one correspondence with the plurality of target phases; and determining a workout duration ratio of a plurality of workout types in each target workout type set in the plurality of target workout type sets, to obtain a plurality of target duration ratios that are in a one-to-one correspondence with the plurality of target workout type sets.

In some possible implementations, determining the target workout item set of the corresponding target phase based on the first workout capability and each target workout type set includes: obtaining a first score based on the first workout capability, where the first score is a score of the first workout capability; determining a first workout difficulty based on a mapping relationship between a workout capability score and a workout difficulty, where the first workout difficulty is a workout difficulty associated with the first score; and determining, based on each target workout type set and the first workout difficulty, a target workout item set of a target phase corresponding to each target workout type set, where the target workout item set of the target phase corresponding to each target workout type set includes a workout item that is of each workout type in each target workout type set and whose workout difficulty is the first workout difficulty.

In some possible implementations, the method includes: obtaining a completion degree of the first target workout plan and/or feedback of the user on the first target workout plan, and determining a second target workout plan.

In some possible implementations, obtaining the completion degree of the first target workout plan includes: obtaining actual completion duration of each workout item in each of the plurality of target workout item sets; determining planned duration of each workout item in each target workout item set based on the plurality of pieces of target duration and the plurality of workout duration ratios; determining, based on actual completion duration and planned duration of workout items of a same type in each target workout item set, completion degrees of the workout items of the same type in each target workout item set; and determining, based on completion degrees of the workout items of the same type in each target workout item set, a workout completion degree of the target phase corresponding to each target workout item set.

According to a third aspect, this application provides an apparatus. The apparatus may include modules that are in one-to-one correspondence with methods/operations/steps/actions described in the first aspect or the second aspect. The modules may be implemented by a hardware circuit, software, or a combination of a hardware circuit and software.

In a design, the apparatus may include a processing module and a communication module. The communication module is configured to perform an information obtaining action in the method described in the second aspect, and the processing module is configured to perform a processing action in the method described in the first aspect or the second aspect.

In a design, the apparatus may be an electronic device, or may be an apparatus, a module, a circuit, a chip, or the like configured in an electronic device, or an apparatus that can be used together with the electronic device.

According to a fourth aspect, an apparatus is provided. The apparatus includes a processor and a storage medium. The storage medium stores instructions. When the instructions are run by the processor, the method according to any one of the first aspect or the possible implementations of the first aspect is implemented.

According to a fifth aspect, an apparatus is provided. The apparatus includes a processor. The processor is configured to process data and/or information, to implement the method according to any one of the first aspect or the possible implementations of the first aspect, or implement the method according to any one of the second aspect or the possible implementations of the second aspect.

In a design, the apparatus may be an electronic device, or may be an apparatus, a module, a circuit, a chip, or the like configured in an electronic device, or an apparatus that can be used together with the electronic device.

Optionally, the apparatus may further include a memory. The memory is configured to store a program or instructions. The processor is configured to run the program or the instructions, to implement the method according to any one of the first aspect or the possible implementations of the first aspect, or implement the method according to any one of the second aspect or the possible implementations of the second aspect.

Optionally, the apparatus may further include a transceiver circuit or an input/output interface.

According to a sixth aspect, a chip is provided. The chip includes a processor. The processor is configured to run a program or instructions, to implement the method according to any one of the first aspect or the possible implementations of the first aspect, or implement the method according to any one of the second aspect or the possible implementations of the second aspect.

Optionally, the chip may further include a memory, and the memory is configured to store the program or the instructions. Optionally, the chip may further include a transceiver circuit or an input/output interface.

According to a seventh aspect, a computer-readable storage medium is provided. The computer-readable storage medium includes instructions. When the instructions are run by a processor, the method according to any one of the first aspect or the possible implementations of the first aspect is implemented, or the method according to any one of the second aspect or the possible implementations of the second aspect is implemented.

According to an eighth aspect, a computer program product is provided. The computer program product includes computer program code or instructions. When the computer program code or the instructions are run, the method in the first aspect or any possible implementation of the first aspect is implemented, or the method in the second aspect or any possible implementation of the second aspect is implemented.

## BRIEF DESCRIPTION OF DRAWINGS

FIG. 1 is a diagram of a structure of an electronic device;
FIG. 2 is a block diagram of a software structure of an electronic device according to an embodiment of this application;
FIG. 3 is a schematic flowchart of a health plan formulation method according to an embodiment of this application;
FIG. 4 is a diagram of determining a fat proportion model based on a group category to which a user belongs according to an embodiment of this application;
FIG. 5 is an example diagram of a user interface according to an embodiment of this application;
FIG. 6 is a schematic flowchart of a health plan formulation method according to another embodiment of this application;
FIG. 7 is an example diagram of determining a metabolism condition of a user according to an embodiment of this application;
FIG. 8 is a diagram of a structure of an apparatus according to an embodiment of this application; and
FIG. 9 is a diagram of a structure of an apparatus according to another embodiment of this application.

## DESCRIPTION OF EMBODIMENTS

The following describes technical solutions of embodiments in this application with reference to accompanying drawings.

Terms used in the following embodiments are merely intended to describe specific embodiments, but are not intended to limit this application. The terms "one", "a", "the", "the foregoing", "this", and "the one" of singular forms used in this specification and the appended claims of this application are also intended to include expressions such as "one or more", unless otherwise specified in the context clearly. It should be further understood that in the following embodiments of this application, "at least one" and "one or more" mean one, two, or more. The term "and/or" is used to describe an association relationship between associated objects and represents that three relationships may exist. For example, A and/or B may represent the following cases: Only A exists, both A and B exist, and only B exists, where A and B may be singular or plural. The character "/" generally indicates an "or" relationship between the associated objects.

Reference to "one embodiment", "some embodiments", or the like described in this specification means that a specific feature, structure, or characteristic described with reference to the embodiment is included in one or more embodiments of this application. Therefore, statements such as "in an embodiment", "in some embodiments", "in some other embodiments", and "in other embodiments" that appear at different places in this specification do not necessarily mean referring to a same embodiment. Instead, the statements mean "one or more but not all of embodiments", unless otherwise specifically emphasized in another manner. The terms "include", "contain", "have", and their variants all mean "include but are not limited to", unless otherwise specifically emphasized in another manner.

An electronic device in embodiments of this application may be a mobile phone, a tablet computer, a wearable electronic device (for example, a smartwatch) having a wireless communication function, a laptop (Laptop), or the like. It should be further understood that, in some embodiments, the electronic device may not be the foregoing portable electronic device, but a desktop computer.

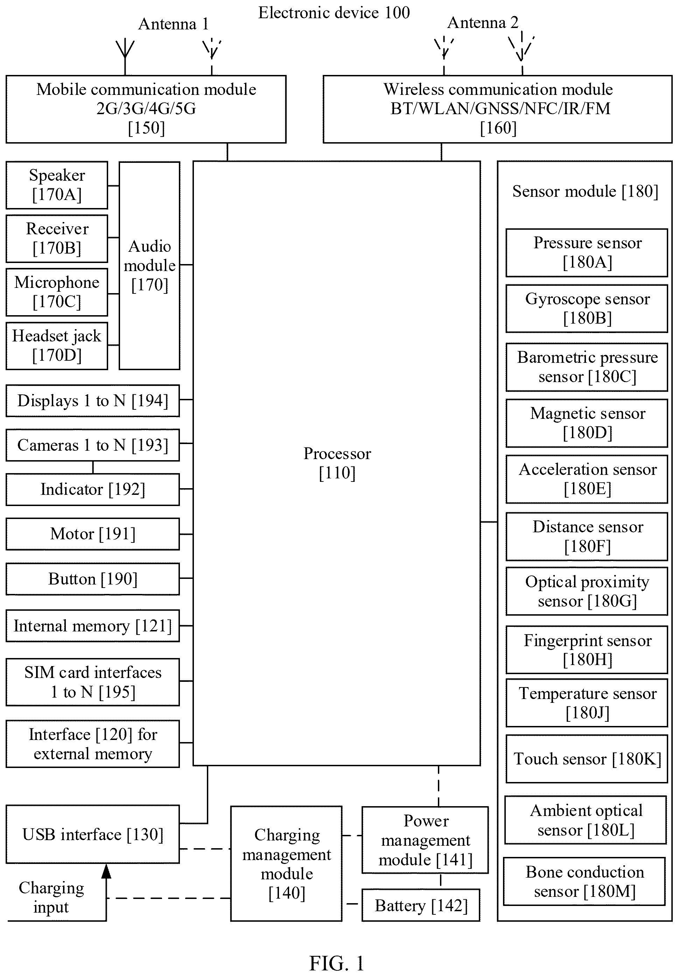

For example, FIG. 1 is a diagram of a structure of an electronic device 100. The electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identification module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in embodiments of this application does not constitute a specific limitation on the electronic device 100. In some other embodiments of this application, the electronic device 100 may include more or fewer components than those shown in the figure, or some components may be combined, or some components may be split, or different component arrangements may be used. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may be a nerve center and a command center of the electronic device 100. The controller may generate an operation control signal based on an instruction operation code and a time sequence signal, to complete control of instruction reading and instruction execution.

A memory may be further disposed in the processor 110, and is configured to store instructions and data. In some embodiments, the memory in the processor 110 is a cache memory. The memory may store instructions or data just used or cyclically used by the processor 110. If the processor 110 needs to use the instructions or the data again, the processor may directly invoke the instructions or the data from the memory. This avoids repeated access, reduces waiting time of the processor 110, and improves system efficiency.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/-transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 100 may be configured to cover one or more communication frequency bands. Different antennas may be further reused, to improve antenna utilization. For example, the antenna 1 may be reused as a diversity antenna of a wireless local area network. In some other embodiments, the antenna may be used in combination with a tuning switch.

The mobile communication module 150 may provide a wireless communication solution that is applied to the electronic device 100 and that includes 2G/3G/4G/5G or the like. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1. In some embodiments, at least some functional modules in the mobile communication module 150 may be disposed in the processor 110. In some embodiments, at least some functional modules of the mobile communication module 150 may be disposed in a same device as at least some modules of the processor 110.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal by an audio device (which is not limited to the speaker 170A, the receiver 170B, or the like), or displays an image or a video by the display 194. In some embodiments, the modem processor may be an independent component. In some other embodiments, the modem processor may be independent of the processor 110, and is disposed in a same device as the mobile communication module 150 or another functional module.

The wireless communication module 160 may provide a wireless communication solution that is applied to the electronic device 100, and that includes a wireless local area network (wireless local area networks, WLAN) (for example, a wireless fidelity (wireless fidelity, Wi-Fi) network), Bluetooth (bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more components integrating at least one communication processor module. The wireless communication module 160 receives an electromagnetic wave by the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some embodiments, the antenna 1 and the mobile communication module 150 in the electronic device 100 are coupled, and the antenna 2 and the wireless communication module 160 in the electronic device 100 are coupled, so that the electronic device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (beidou navigation satellite system, BDS), a quasi-zenith satellite system (quasi-zenith satellite system, QZSS), and/or a satellite based augmentation system (satellite based augmentation systems, SBAS).

The electronic device 100 may implement a display function through the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs, which execute program instructions to generate or change display information.

The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light emitting diode (active-matrix organic light emitting diode, AMOLED), a flexible light-emitting diode (flexible light-emitting diode, FLED), a mini-LED, a micro-LED, a micro-OLED, a quantum dot light emitting diode (quantum dot light emitting diode, QLED), or the like. In some embodiments, the electronic device 100 may include one or N displays 194, where N is a positive integer greater than 1.

The electronic device 100 may implement a photographing function through the camera 193, the ISP, the video codec, the GPU, the display 194, the application processor and the like.

The ISP is configured to process data fed back by the camera 193. For example, during photographing, a shutter is pressed, and light is transmitted to a photosensitive element of the camera through a lens. An optical signal is converted into an electrical signal, and the photosensitive element of the camera transmits the electrical signal to the ISP for processing, to convert the electrical signal into a visible image. The ISP may further perform algorithm optimization on noise, brightness, and complexion of the image. The ISP may further optimize parameters such as exposure and a color temperature of a photographing scenario. In some embodiments, the ISP may be disposed in the camera 193.

The camera 193 is configured to capture a static image or a video. An optical image of an object is generated through the lens, and is projected onto the photosensitive element. The photosensitive element may be a charge coupled device (charge coupled device, CCD) or a complementary metal-oxide-semiconductor (complementary metal-oxide-semiconductor, CMOS) phototransistor. The light-sensitive element converts an optical signal into an electrical signal, and then transmits the electrical signal to the ISP to convert the electrical signal into a digital image signal. The ISP outputs the digital image signal to the DSP for processing. The DSP converts the digital image signal into an image signal in a standard format such as RGB or YUV. In some embodiments, the electronic device 100 may include one or N cameras 193, where N is a positive integer greater than 1.

The digital signal processor is configured to process a digital signal, and may process another digital signal in addition to the digital image signal. For example, when the electronic device 100 selects a frequency, the digital signal processor is configured to perform Fourier transformation on frequency energy.

The video codec is configured to compress or decompress a digital video. The electronic device 100 may support one or more video codecs. In this way, the electronic device 100 may play back or record videos in a plurality of coding formats, for example, moving picture experts group (moving picture experts group, MPEG)-1, MPEG-2, MPEG-3, and MPEG-4.

The NPU is a neural-network (neural-network, NN) computing processor, simulates a biological neural network structure such as a transmission mode between neurons in a human brain to perform rapid processing on input information, and may further perform continuous self-learning. Applications such as intelligent cognition of the electronic device 100 may be implemented through the NPU, for example, image recognition, facial recognition, speech recognition, and text understanding.

The external memory interface 120 may be used to connect to an external storage card, for example, a micro SD card, to extend a storage capability of the electronic device 100. The external memory card communicates with the processor 110 through the external memory interface 120, to implement a data storage function. For example, files such as music and videos are stored in the external storage card.

The internal memory 121 may be configured to store computer-executable program code. The executable program code includes instructions. The processor 110 runs the instructions stored in the internal memory 121, to perform various function applications of the electronic device 100 and data processing. The internal memory 121 may include a program storage area and a data storage area. The program storage area may store an operating system, an application required by at least one function (for example, a voice playing function or an image playing function), and the like. The data storage area may store data (such as audio data and an address book) created during use of the electronic device 100, and the like. In addition, the internal memory 121 may include a high-speed random access memory, or may include a nonvolatile memory, for example, at least one magnetic disk storage device, a flash memory, or a universal flash storage (universal flash storage, UFS).

The electronic device 100 may implement an audio function, for example, music playing and recording, through the audio module 170, the speaker 170A, the receiver 170B, the microphone 170C, the headset jack 170D, the application processor, and the like.

The audio module 170 is configured to convert digital audio information into an analog audio signal for output, and is also configured to convert analog audio input into a digital audio signal. The audio module 170 may be further configured to code and decode an audio signal. In some embodiments, the audio module 170 may be disposed in the processor 110, or some functional modules in the audio module 170 are disposed in the processor 110.

The speaker 170A, also referred to as a "loudspeaker", is configured to convert an audio electrical signal into a sound signal. The electronic device 100 may be used to listen to music or answer a call in a hands-free mode over the speaker 170A.

The receiver 170B, also referred to as an "earpiece", is configured to convert an audio electrical signal into a sound signal. When a call is answered or speech information is received through the electronic device 100, the receiver 170B may be put close to a human ear to listen to a voice.

The microphone 170C, also referred to as a "mike" or a "mic", is configured to convert a sound signal into an electrical signal. When making a call or sending a voice message, a user may make a sound near the microphone 170C through the mouth of the user, to input a sound signal to the microphone 170C. At least one microphone 170C may be disposed in the electronic device 100. In some other embodiments, two microphones 170C may be disposed in the electronic device 100, to capture a sound signal and implement a noise reduction function. In some other embodiments, three, four, or more microphones 170C may alternatively be disposed in the electronic device 100, to capture a sound signal, implement noise reduction, and identify a sound source, so as to implement a directional recording function and the like.

The pressure sensor 180A is configured to sense a pressure signal, and can convert the pressure signal into an electrical signal. In some embodiments, the pressure sensor 180A may be disposed on the display 194. There are a plurality of types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 100 determines pressure intensity based on the change in the capacitance. When a touch operation is performed on the display 194, the electronic device 100 detects intensity of the touch operation through the pressure sensor 180A. The electronic device 100 may also calculate a touch location based on a detection signal of the pressure sensor 180A. In some embodiments, touch operations that are performed in a same touch position but have different touch operation intensity may correspond to different operation instructions. For example, when a touch operation whose touch operation intensity is greater than or equal to a first pressure threshold acts on an alarm clock application icon, an instruction for creating an alarm clock is executed.

The fingerprint sensor 180H is configured to collect a fingerprint. The electronic device 100 may use a feature of the collected fingerprint to implement fingerprint-based unlocking, application lock access, fingerprint-based photographing, fingerprint-based call answering, and the like. For example, when a mobile phone detects a touch operation of a user on a lock screen interface, the mobile phone may collect fingerprint information of the user through the fingerprint sensor 180H, and match the collected fingerprint information with fingerprint information preset in the mobile phone. If matching succeeds, the mobile phone may enter a non-lock screen interface from the lock screen interface.

The touch sensor 180K is also referred to as a touch panel. The touch sensor 180K may be disposed in the display 194, and the touch sensor 180K and the display 194 form a touchscreen, which is also referred to as a "touchscreen". The touch sensor 180K is configured to detect a touch operation performed on or near the touch sensor. The touch sensor may transfer the detected touch operation to the application processor to determine a type of the touch event. A visual output related to the touch operation may be provided through the display 194. In some other embodiments, the touch sensor 180K may also be disposed on a surface of the electronic device 100 at a location different from that of the display 194.

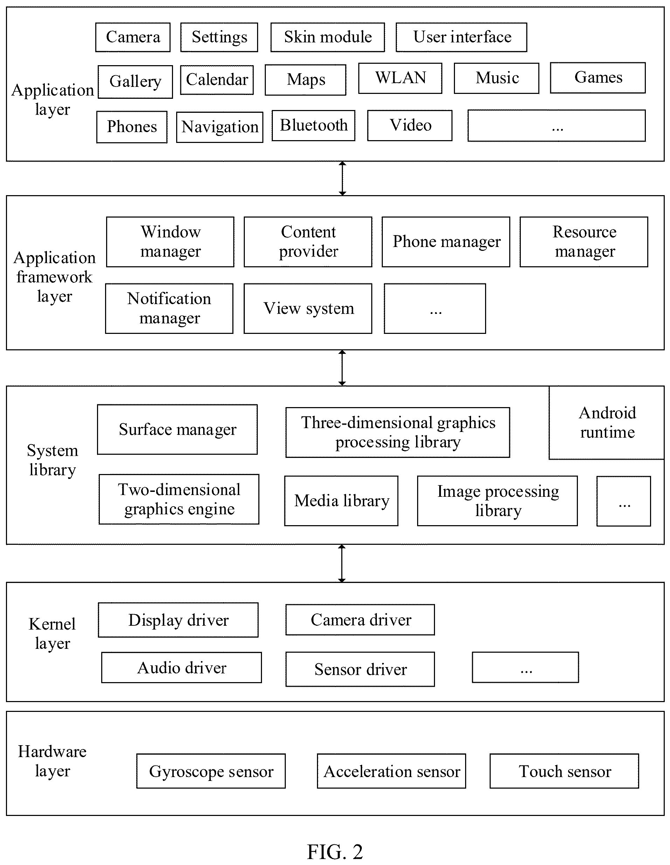

FIG. 2 is a block diagram of a software structure of the electronic device 100 according to an embodiment of this application. In a layered architecture, software is divided into several layers, and each layer has a clear role and task. The layers communicate with each other through a software interface. In some embodiments, a software system of the electronic device is divided into four layers: an application layer, an application framework layer, a runtime (runtime) and system library, and a kernel layer from top to bottom. The application layer may include a series of application packages.

As shown in FIG. 2, the application layer may include Camera, Settings, a skin module, a user interface (user interface, UI), a third-party application, and the like. The third-party application may include Gallery, Calendar, Phones, Maps, Navigation, WLAN, Bluetooth, Music, Videos, Messages, and the like.

The application framework layer provides an application programming interface (application programming interface, API) and a programming framework for an application at the application layer. The application framework layer may include some predefined functions.

As shown in FIG. 2, the application framework layer may include a window manager, a content provider, a view system, a phone manager, a resource manager, a notification manager, and the like.

The window manager is configured to manage a window program. The window manager may obtain a size of the display, determine whether there is a status bar, perform screen locking, take a screenshot, and the like. The content provider is configured to: store and obtain data, and enable the data to be accessed by an application. The data may include a video, an image, an audio, calls that are made and answered, a browsing history and bookmarks, an address book, and the like.

The view system includes a visual control, like a control for displaying a text or a control for displaying a picture, for example, displaying indication information for prompting a virtual shutter button in this embodiment of this application. The view system may be configured to construct an application. A display interface may include one or more views. For example, a display interface including an SMS message notification icon may include a text display view and an image display view.

The phone manager is configured to provide a communication function for the electronic device 100, for example, management of a call status (including answering, declining, or the like).

The resource manager provides various resources such as a localized character string, an icon, an image, a layout file, and a video file for an application.

The notification manager enables an application to display notification information in a status bar, and may be configured to convey a notification message. The notification manager may automatically disappear after a short pause without requiring a user interaction. For example, the notification manager is configured to notify download completion, give a message notification, and the like. The notification manager may alternatively be a notification that appears in a top status bar of the system in a form of a graph or a scroll bar text, for example, a notification of an application that is run on a background, or may be a notification that appears on the screen in a form of a dialog window. For example, text information is displayed in the status bar, an announcement is given, the electronic device vibrates, or the indicator light blinks.

The runtime includes a core library and a virtual machine. The runtime is responsible for scheduling and management of the software system.

The core library includes two parts: a function that needs to be invoked by a programming language, and a system core library.

The application layer and the application framework layer run on the virtual machine. The virtual machine executes java files of the application layer and the application framework layer as binary files. The virtual machine is configured to implement functions such as object lifecycle management, stack management, thread management, security and exception management, and garbage collection.

The system library may include a plurality of functional modules, for example, a surface manager (surface manager), a media library (media library), a three-dimensional graphics processing library (for example, OpenGL ES), and a 2D graphics engine (for example, SGL).

The surface manager is configured to manage a display subsystem and provide fusion of 2D and 3D layers for a plurality of applications.

The media library supports playback and recording in a plurality of commonly used audio and video formats, and static image files. The media library may support a plurality of audio and video coding formats, for example, MPEG-4, H.264, MP3, AAC, AMR, JPG, and PNG.

The three-dimensional graphics processing library is configured to implement three-dimensional graphics drawing, image rendering, composition, layer processing, and the like.

The 2D graphics engine is a drawing engine for 2D drawing.

In addition, the system library may further include a status monitoring service module, for example, a physical status identification module, configured to analyze and identify a user gesture; and a sensor service module, configured to monitor sensor data uploaded by various sensors at a hardware layer, to determine a physical status of the electronic device 100.

The kernel layer is a layer between hardware and software. The kernel layer includes at least a display driver, a camera driver, an audio driver, and a sensor driver.

The hardware layer may include various types of sensors, for example, various types of sensors described in FIG. 1, and an acceleration sensor, a gyroscope sensor, a touch sensor, and the like in embodiments of this application.

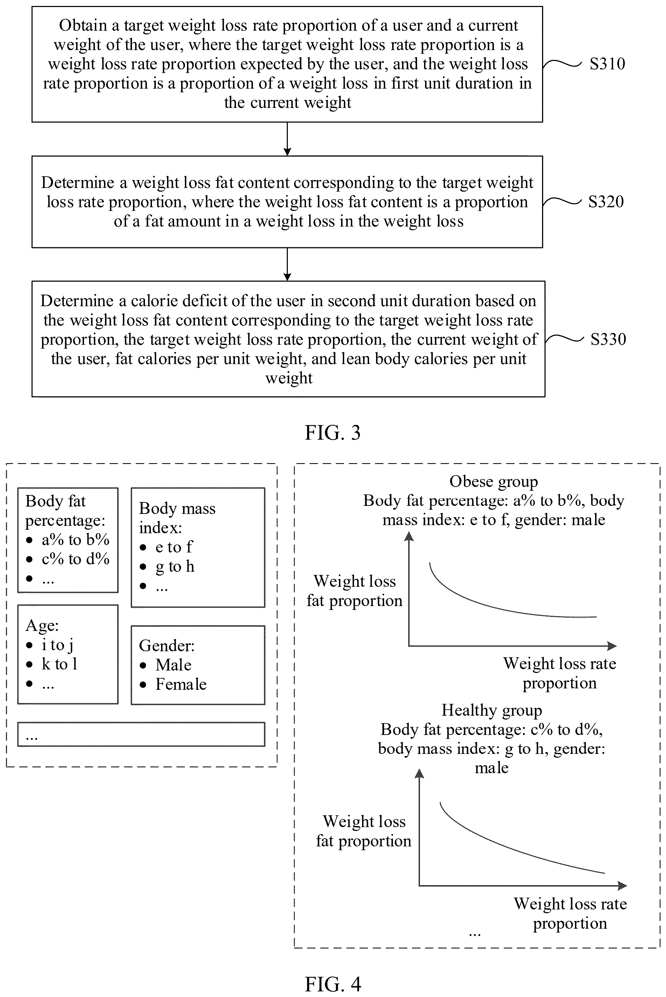

FIG. 3 is a schematic flowchart of a health plan formulation method according to an embodiment of this application. As shown in FIG. 3, the method includes S310, S320, and S330. The method may be performed by an electronic device. The electronic device may be a terminal device, for example, a terminal device such as a mobile phone or a tablet computer, may be a wearable device, or may be a cloud server. An example of a structure of the electronic device is shown in FIG. 1 and/or FIG. 2.

S310: Obtain a target weight loss rate proportion of a user and a current weight of the user, where the target weight loss rate proportion is a weight loss rate proportion expected by the user, and the weight loss rate proportion is a proportion of a weight loss in first unit duration in the current weight.

For example, the first unit duration may be a day, a week, a month, a year, or the like.

For example, the weight loss rate proportion is a percentage of the weight loss in the first unit duration in the current weight. For example, the weight loss rate may be 1% per week.

For example, when the user expects to lose the current weight by 1% in one week, the first unit duration is one week, and the target weight loss rate of the user is 1%.

S320: Determine a weight loss fat content corresponding to the target weight loss rate proportion, where the weight loss fat content is a proportion of a fat amount in a weight loss in the weight loss.

For example, the weight loss fat content is a percentage of a fat amount in the weight loss in the weight loss. For example, when the weight loss is 1 kilogram (kg) and a fat content loss is 0.5 kg, the weight loss fat content is a percentage value of 0.5 kg to 1 kg, that is, 50%.

Generally, there is a mapping relationship between the weight loss rate proportion and the weight loss fat content. For example, a larger weight loss rate proportion indicates a smaller weight loss fat proportion, that is, the weight loss fat proportion is inversely proportional to the weight loss rate proportion.

In this step, in an example, the weight loss fat content corresponding to the target weight loss rate proportion may be determined based on the mapping relationship between the weight loss rate proportion and the weight loss fat content.

S330: Determine a calorie deficit of the user in second unit duration based on the weight loss fat content corresponding to the target weight loss rate proportion, the target weight loss rate proportion, the current weight of the user, fat calories per unit weight, and lean body calories per unit weight.

In this step, the lean body may be understood as a general term of a set of components other than fat in the human body.

For example, the second unit duration may be a day, a week, a month, a year, or the like.

For example, the unit weight may be grams (g), kg, half-kilograms, kilograms, or the like.

For example, the weight loss fat content corresponding to the target weight loss rate proportion, the target weight loss rate proportion, the current weight of the user, the fat calories per unit weight, the lean body calories per unit weight, and the calorie deficit of the user in the second unit duration satisfy the following relation

$$g=(w*v*p*k1+w*v*(1-p)*k2)/t.$$

g represents the calorie deficit in the second unit duration, w represents the current weight of the user, v represents the target weight loss rate proportion, p represents the weight loss fat content corresponding to the target weight loss rate proportion, k1 represents the fat calories per unit weight, k2 represents the lean body calories per unit weight, and t represents a ratio of the first unit duration to the second unit duration.

It may be understood that the foregoing relation is merely an example relation for calculating the calorie deficit based on the weight loss fat content corresponding to the target weight loss rate proportion, the target weight loss rate proportion, the current weight of the user, the fat calories per unit weight, and the lean body calories per unit weight. Any relation obtained by transforming the foregoing relation should fall within the scope of this step.

It may be understood that a principle of this step is as follows: A target weight loss may be learned based on the current weight and the target weight loss rate proportion in the first unit duration, where the target weight loss is a weight that the user expects to lose in the first unit duration. A fat weight in the target weight loss, that is, the fat weight included in the weight that the user expects to lose in the first unit duration, may be learned based on the target weight loss and the weight loss fat content corresponding to the target weight loss rate proportion. Fat calories that need to be lost in the first unit duration may be learned based on the fat weight in the target weight loss and the fat calories per unit weight. A weight loss lean body content corresponding to the target weight loss rate proportion may be learned based on the weight loss fat content corresponding to the target weight loss rate proportion, where the weight loss lean body content is a proportion of the lean body in the weight that is lost in the first unit duration in the weight loss. A lean body weight in the target weight loss, that is, the lean body weight included in the weight that the user expects to lose in the first unit duration, may be learned based on the target weight loss and the weight loss lean body content corresponding to the target weight loss rate proportion. Lean body calories that need to be lost in the first unit duration may be learned based on the lean body weight in the target weight loss and the lean body calories per unit weight. Total calories that need to be lost in the first unit duration may be learned based on the lean body calories that need to be lost in the first unit duration and the lean body calories that need to be lost in the first unit duration. Calories that need to be lost in the second unit duration, that is, the calorie deficit in the second unit duration, may be learned based on the total calories that need to be lost in the first unit duration and the ratio of the first unit duration to the second unit duration.

It may be understood that any method for determining the calorie deficit based on the weight loss fat content corresponding to the target weight loss rate proportion, the target weight loss rate proportion, the current weight of the user, the fat calories per unit weight, and the lean body calories per unit weight by using the foregoing principle or a related principle that can be unambiguously derived from the foregoing principle shall fall within the scope of this step.

In this embodiment, in an example, the first unit duration may be default duration. For example, the default duration is one week or one month.

For example, the first unit duration may be input by the user, for example, input through a user interface.

For example, weight loss duration may be obtained through calculation based on a target weight expected by the user, the current weight, and a weight loss rate. For example, a value obtained by dividing a difference between the target weight and the current weight by the weight loss rate may be used as the weight loss duration. Optionally, the weight loss rate may also be obtained through calculation based on the weight loss rate proportion.

For example, the target weight of the user may be obtained by inputting by the user. For example, the target weight input by the user through the user interface may be obtained.

For example, the user interface may include a text input control, and the user may input the target weight in the text input control.

For example, the user interface may include a selection control, and the selection control may provide the user with a plurality of weight options. The user may select the target weight through the selection control.

In this embodiment, in an example, the second unit duration may be default duration. For example, the default duration is one week or one month.

For example, the second unit duration may be input by the user, for example, input through the user interface.

In this embodiment, in an example, obtaining the target weight loss rate proportion of the user may include: obtaining a target weight loss rate of the user and the current weight of the user; and determining the target weight loss rate proportion based on the target weight loss rate of the user and the current weight of the user.

For example, determining the target weight loss rate proportion based on the target weight loss rate of the user and the current weight of the user may include: obtaining a ratio of the target weight loss rate of the user to the current weight of the user, to obtain the target weight loss rate proportion.

For example, obtaining the target weight loss rate of the user may include: obtaining the target weight loss rate input by the user. For example, the target weight loss rate input by the user through a control in the user interface is obtained.

For example, the user interface may include the text input control, and the user may input the target weight loss rate in the text input control.

For example, the user interface may include the selection control, and the selection control may provide the user with a plurality of weight loss rate options. The user may select a weight loss rate through the selection control, where the weight loss rate selected by the user may be used as the target weight loss rate input by the user.

For example, obtaining the target weight loss rate of the user may include: obtaining a weight loss mode expected by the user, and determining the target weight loss rate of the user based on a mapping relationship between the weight loss mode of the user and the weight loss rate. Different weight loss modes correspond to different weight loss rates. The weight loss mode expected by the user may be referred to as a target weight loss mode.

The weight loss mode may include at least one of the following modes: a recommendation mode, a fast mode, or a radical mode. Three weight loss rates corresponding to the three modes may increase in sequence.

For example, the weight loss rate corresponding to the fast mode may be between 1.1% and 1.5%.

For example, obtaining the weight loss mode of the user may include: obtaining the target weight loss mode input by the user through a control in the user interface.

For example, the user interface may include the text input control, and the user may input the target weight loss mode in the text input control.

For example, the user interface may include the selection control, and the selection control may provide the user with a plurality of weight loss mode options. The user may select a weight loss mode through the selection control, where the weight loss mode selected by the user may be used as the target weight loss mode input by the user.

Optionally, in a scenario in which the user interface includes the selection control used to select the weight loss mode, the user interface may further include a weight loss rate corresponding to each selectable weight loss mode, to provide a reference for the user to select the weight loss mode.

For example, obtaining the target weight loss rate proportion of the user may include: obtaining the target weight loss rate proportion input by the user. For example, the target weight loss rate proportion input by the user through the user interface is obtained.

For example, the user interface may include the text input control, and the user may input the target weight loss rate proportion in the text input control.

For example, the user interface may include the selection control, and the selection control may provide the user with a plurality of weight loss rate proportion options. The user may select a weight loss rate proportion through the selection control, where the weight loss mode selected by the user may be used as the target weight loss rate proportion input by the user.

Optionally, in a scenario in which the user interface includes the selection control used to select the weight loss rate, the user interface may further include a weight loss rate and/or a weight loss mode corresponding to each selectable weight loss rate proportion, to provide a reference for the user to select the weight loss rate proportion.

For example, obtaining the current weight of the user may include: obtaining the current weight input by the user through a control in the user interface.

For example, the user interface may include the text input control, and the user may input the current weight in the text input control.

For example, the user interface may include the selection control, and the selection control may provide the user with a plurality of weight options. The user may select a weight through the selection control, where the weight selected by the user may be used as the current weight input by the user.

For example, obtaining the current weight of the user may include: receiving a weight obtained by measuring the user by a weight detection device. An example of the weight detection device is a body fat scale.

For example, the electronic device establishes a connection to the weight detection device, then receives, from the weight detection device, the weight of the user that is currently measured by the weight detection device in real time, or receives, from the weight detection device, a weight measured by the user by using the weight detection device last time, and uses the received weight value as the current weight of the user.

For example, determining the weight loss fat content corresponding to the target weight loss rate proportion includes: determining a group category to which the user belongs; determining a first mapping relationship from a plurality of mapping relationships based on the group category to which the user belongs, where the first mapping relationship is a mapping relationship corresponding to the group category to which the user belongs in the plurality of mapping relationships, the plurality of mapping relationships are in a one-to-one correspondence with a plurality of group categories, and each mapping relationship in the plurality of mapping relationships indicates a mapping relationship between a fat proportion in a weight loss of a user of a corresponding group category and a weight loss proportion of the user of the corresponding group category in the first unit duration; and determining, based on the first mapping relationship, the weight loss fat content corresponding to the target weight loss rate proportion.

In this embodiment, the mapping relationship between the fat proportion in the weight loss of the user of the corresponding group category and the weight loss proportion of the user of the corresponding group category in the first unit duration may be referred to as a weight loss fat proportion model.

For example, the group category may include at least one of the following categories: an obese group, a fitness group, and a lean group.

In this step, at least one piece of the following information of users of different group categories is different: a maximum oxygen uptake, a body fat percentage, an age, a gender, a current weight, or a body mass index (body mass index, BMI).

For example, the group category may be represented by a name of the group category. For example, the name of the group category includes "obese group", "fitness group", or "lean group".

For example, the group category may be represented by feature information of the user in the group category. For example, a high body fat percentage, a high BMI, and a male may represent the obese group. For another example, a low body fat percentage, a high BMI, and a male may represent the fitness group.

For example, the group category may be represented by a range to which a feature information value of the user in the group category belongs. For example, a body fat percentage of a1% to b1%, a BMI of c1% to d1%, and a male may represent the obese group. For another example, a body fat percentage of a2% to b2%, a BMI of c2% to d2%, and a male may represent the fitness group.

For example, determining the group category to which the user belongs may include: obtaining information that is input by the user and that can represent the group category, and determining, based on the information, the group category to which the user belongs.

For example, the user interface may include the text input control, and the user may input the information that represents the group category in the text input control.

For example, the user interface may include the selection control. The selection control may provide the user with information options representing different group categories. The user may select, through the selection control, information representing the different group categories. The information selected by the user may be used as information that is input by the user and that represents the different group categories.

For example, when the name of the group category to which the user belongs is obtained, and the mapping relationship between the weight loss rate proportion and the weight loss fat content is associated with the name of the group category, the first mapping relationship may be determined based on the name of the group category to which the user belongs.

For example, when the feature information of the user is obtained, and the mapping relationship between the weight loss rate proportion and the weight loss fat content is associated with the name of the group category, the name of the group category to which the user belongs needs to be first determined based on a mapping relationship between the feature information of the user and the group category. Then, the first mapping relationship is determined based on the name of the group category to which the user belongs.

For example, when the feature information of the user is obtained, and the mapping relationship between the weight loss rate proportion and the weight loss fat content is associated with a feature of the user, the first mapping relationship may be determined based on the feature information of the user.

For example, when the feature information of the user is obtained, and the mapping relationship between the weight loss rate proportion and the weight loss fat content is associated with a range to which a feature value belongs, a range to which a feature value of the user belongs may be first determined based on a mapping relationship between the feature information of the user and the range to which the feature value belongs. Then, the first mapping relationship is determined based on the range to which the feature value of the user belongs.

For example, when the feature information of the user is obtained, and the mapping relationship between the weight loss rate proportion and the weight loss fat content is associated with the name of the group category, the range to which the feature value of the user belongs may be first determined based on the mapping relationship between the feature information of the user and the range to which the feature value belongs. Then, the name of the group category to which the user belongs is determined based on the mapping relationship between the range to which the feature value of the user belongs and the group category. Finally, the first mapping relationship is determined based on the name of the group category to which the user belongs.

FIG. 4 is a diagram of determining a fat proportion model based on a group category to which a user belongs. As shown in FIG. 4, a body fat percentage may be divided into several ranges such as a1% to b1% and a2% to b2%. A BMI may be divided into several ranges such as c1 to d1 and c2 to d2. An age may be divided into several ranges such as e1 to f1 and e2 to f2. A gender is divided into male and female.

The body fat percentage a1% to b1%, the BMI c1 to d1, the age e1 to f1, and the male gender are associated with a fat proportion model. The body fat percentage a2% to b2%, the BMI c2 to d2, the age e2 to f2, and the male gender are associated with another weight loss fat proportion model.

It may be understood that, in FIG. 4, information "body fat percentage a1% to b1%, BMI c1 to d1, age e1 to f1, and male gender" representing a group category may be replaced with a name "obese group" of a corresponding group category. Information "body fat percentage a2% to b2%, BMI c2 to d2, age e2 to f2, and male gender" representing a group category may be replaced with a name "fitness group" of a corresponding group category.

It may be understood that, in FIG. 4, information "body fat percentage a1% to b1%, BMI c1 to d1, age e1 to f1, and male gender" representing a group category may be replaced with information "high body fat percentage, high BMI, old, and male" representing a group category. Information "body fat percentage a2% to b2%, BMI c1 to d1, age e2 to f2, and male gender" representing a group category may be replaced with information "low body fat percentage, high BMI, young, and male" representing the group category.

As shown in FIG. 4, when the obtained user information is "body fat percentage a, BMI c, age e, and male gender", and a is between a1 and b1, c is between c1 and d1, and e is between e1 and f1, the first mapping relationship may be determined based on the user information as a weight loss fat proportion model located in an upper part in two weight loss fat proportion models shown in FIG. 4.

In this embodiment, optionally, the target weight loss rate proportion may be a weight loss rate proportion range, and the weight loss rate proportion range is also referred to as a weight loss rate proportion interval.

In this scenario, a calorie deficit corresponding to each endpoint value may be obtained based on each endpoint value in two endpoint values of the weight loss rate proportion range according to the foregoing method. A calorie range in which two calorie deficits in a one-to-one correspondence with the two endpoint values are used as an upper limit of the interval and a lower limit of the interval is used as the calorie deficit corresponding to the target weight loss rate. The calorie deficit is a range. A larger value of the two calorie deficits is the upper limit of the interval, and a smaller value is the lower limit of the interval.

In this embodiment, after the calorie deficit of the user in the second unit duration is obtained, the calorie deficit may be output. For example, the calorie deficit may be output through the user interface.

Optionally, when the calorie deficit is output through the user interface, all or some of the information for obtaining the calorie deficit may be further output.

Optionally, in this embodiment, the method may further include the following steps: determining a diet plan and a workout plan of the user in the second unit duration based on the calorie deficit and basal metabolism calories of the user in the second unit duration.

For example, the basal metabolism calories of the user in the second unit duration may be directly input by the user, or may be calculated based on other information of the user.

For example, the diet plan of the user in the second unit duration may include the following content: total calories that can be ingested in the second unit duration, which is denoted as first calories; and total calories that should be consumed in the second unit duration, which is denoted as second calories. A difference between the first calories and the second calories is equal to the calorie deficit.

It may be understood that, if the calorie deficit is a range, the first calories and the second calories in the diet plan may also be ranges.

For example, the diet plan of the user in the second unit duration may further include the following content: a quantity of daily meals, and calories that can be ingested in each meal. It may be understood that, if the calorie deficit is a range, the calories that can be ingested in each meal may also be a range.

For example, the workout plan may include a workout type, may further include a workout item included in each workout type, and may further include duration information of a workout item of each workout type. The duration information may be absolute duration, for example, one hour; or may be relative duration, for example, a duration ratio of workout items of different workout types.

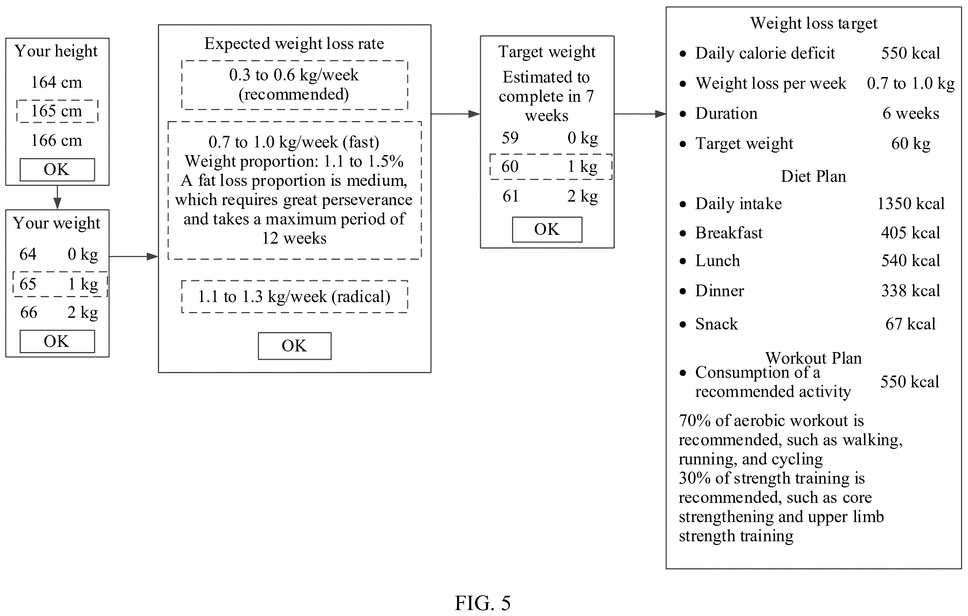

FIG. 5 is an example diagram of a user interface according to an embodiment of this application. As shown on the left of a dashed line in FIG. 5, a user information input user interface may output a plurality of height values for the user to select, may output a plurality of weight values for the user to select, may provide a plurality of weight loss rates for the user to select, may provide a plurality of weight loss rate proportions for the user to select, may provide a plurality of weight loss modes for the user to select, and may provide a plurality of target weights for the user to select.

As shown on the right of the dashed line in FIG. 5, a calorie deficit user interface may include the following three parts: a weight loss target, a diet plan, and a workout plan.

The weight loss target part may include the following content: a daily calorie deficit, a weekly weight loss rate, weight loss duration, and a target weight.

The diet plan part may include the following content: total calories that can be ingested per day, calories that can be ingested for breakfast, calories that can be ingested for lunch, calories that can be ingested for dinner, and calories that can be ingested for snack.

The workout plan part may include the following content: calories that can be consumed by recommended activities, and a recommended workout type determined based on the calories that can be consumed by the recommended activities. For example, when the calories that can be consumed by the recommended activity are 550 kcal, the recommended workout type is: recommending 70% of aerobic workout such as walking, running, and cycling, and/or 30% of strength training such as core strengthening and upper limb strength training.

In some other embodiments of this application, if the method shown in FIG. 3 is performed by a cloud server, the terminal device, for example, a mobile phone, may obtain related user information and send the related user information to the cloud server. After the cloud server performs the method in the embodiment shown in FIG. 3 to obtain the calorie deficit or even other information that can be output to the user, the cloud server sends the calorie deficit and the related information to the terminal device. The terminal device outputs the calorie deficit and the related information through the user interface.

Optionally, the terminal device may further send the calorie deficit and the related information to a wearable device, and display the calorie deficit and the related information to the user through the wearable device, so that convenience of browsing the calorie deficit and the related information by the user can be further improved.

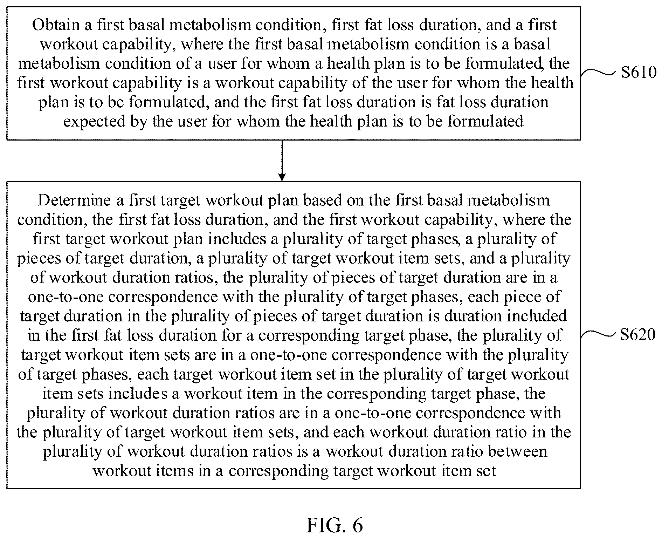

FIG. 6 is a schematic flowchart of a health plan formulation method according to another embodiment of this application. As shown in FIG. 6, the method may include S610 and S620.

S610: Obtain a first basal metabolism condition, first fat loss duration, and a first workout capability, where the first basal metabolism condition is a basal metabolism condition of a user for whom a health plan is to be formulated, the first workout capability is a workout capability of the user for whom the health plan is to be formulated, and the first fat loss duration is fat loss duration expected by the user for whom the health plan is to be formulated.

For example, the basal metabolism condition is used to indicate a basal metabolism speed, and different basal metabolism conditions indicate different basal metabolism speeds.

For example, the basal metabolism condition may include high metabolism, standard metabolism, and low metabolism, and basal metabolism speeds corresponding to the three basal metabolism conditions are in descending order.

For example, obtaining the first basal metabolism condition may include: obtaining the first basal metabolism condition input by the user. For example, the first basal metabolism condition is input by the user through a control in a user interface.

For example, the user interface may include a text input control, and the user may input the first basal metabolism condition in the text input control.

For example, the user interface may include a selection control, and the selection control may provide the user with a plurality of basal metabolism condition options. The user may select a basal metabolism condition through the selection control. The basal metabolism condition selected by the user may be used as the first basal metabolism condition input by the user.

For example, the user interface may display a basal metabolism value range corresponding to each basal metabolism condition, so that the user can determine, based on a basal metabolism value of the user, a basal metabolism value range in which the user is located, so that the user can accurately select the basal metabolism condition of the user.

For example, obtaining the first basal metabolism condition may include: obtaining the basal metabolism value input by the user, and determining the first basal metabolism condition of the user based on the basal metabolism value. For example, a basal metabolism value range to which the basal metabolism value input by the user belongs is determined, and a basal metabolism condition corresponding to the basal metabolism value range is determined as the first basal metabolism condition. For ease of description, the basal metabolism value input by the user is referred to as a first basal metabolism value.

For example, obtaining the first basal metabolism value input by the user may include: obtaining the first basal metabolism value input by the user through the control in the user interface.

For example, the user interface may include the text input control, and the user may input the first basal metabolism value in the text input control.

For example, the user interface may include a selection control, and the selection control may provide the user with a plurality of basal metabolism value options. The user may select a basal metabolism value through the selection control. The basal metabolism value selected by the user may be used as the first basal metabolism value input by the user.

For example, obtaining the first basal metabolism condition input by a user may include: obtaining at least one piece of information of a BMI, a gender, and an age of the user and a body fat percentage, and determining the first basal metabolism condition based on the at least one piece of information and the body fat percentage.

For a manner of obtaining the BMI, the gender, and the age of the user, refer to related content in the embodiment shown in FIG. 3. Details are not described herein again.

For example, determining the first basal metabolism condition based on at least one piece of information of the BMI, the gender, and the age and the body fat percentage may include: obtaining at least one piece of information of the BMI, the gender, and the age of the user and the body fat percentage of the user; determining, based on a correspondence between the at least one piece of information and a normal body fat percentage, a normal body fat percentage range associated with the at least one piece of information; and determining a relationship between the body fat percentage of the user and the normal body fat percentage range. If the body fat percentage of the user is higher than the normal body fat percentage range, it may be considered that the first basal metabolism condition is high metabolism. If the body fat percentage of the user is within the normal body fat percentage range, it may be considered that the first basal metabolism condition is standard basal metabolism. If the body fat percentage of the user is lower than the normal body fat percentage range, it may be considered that the first basal metabolism condition is low metabolism.

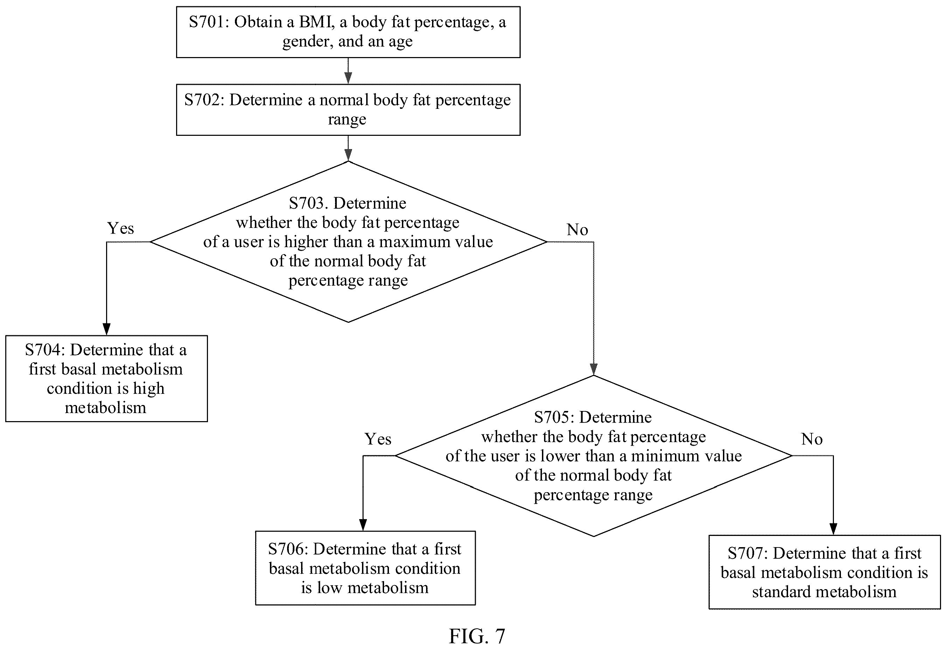

FIG. 7 is a flowchart of determining a first basal metabolism condition based on a body fat percentage and at least one piece of information: a BMI, a gender, and an age. The method shown in FIG. 7 includes S701 to S707.

S701: Obtain a BMI, a gender, an age, and a body fat percentage of a user.

S702: Determine, based on a correspondence between the BMI, the gender, and the age of the user and a normal body fat percentage, a normal body fat percentage range associated with the at least one piece of information.

S703: Determine whether the body fat percentage of the user is higher than a maximum value of the normal body fat percentage range. If the body fat percentage of the user is higher than the maximum value of the normal body fat percentage range, S704 may be performed. If the body fat percentage of the user is not higher than the maximum value of the normal body fat percentage range, S705 may be performed.

S704: Determine that a first basal metabolism condition is high metabolism.

S705: Determine whether the body fat percentage of the user is lower than a minimum value of the normal body fat percentage range. If the body fat percentage of the user is lower than the minimum value of the normal body fat percentage range, S706 is performed. If the body fat percentage of the user is not lower than the minimum value of the normal body fat percentage range, S707 is performed.

S706: Determine that the first basal metabolism condition is low metabolism.

S707: Determine that the first basal metabolism condition is standard metabolism.

Alternatively, on the contrary, it is first determined whether the body fat percentage of the user is less than the minimum value of the normal body fat percentage range. If the body fat percentage of the user is less than the minimum value of the normal body fat percentage range, S706 is performed. If the body fat percentage of the user is not less than the minimum value of the normal body fat percentage range, it is determined whether the body fat percentage of the user is greater than the maximum value of the normal body fat percentage range. If the body fat percentage of the user is greater than the maximum value of the normal body fat percentage range, S705 is performed. If the body fat percentage of the user is not greater than the maximum value of the normal body fat percentage range, S707 is performed.

The following describes a manner of obtaining the first workout capability and the first fat loss duration in S610.

For example, for a manner of obtaining the first weight loss duration in this embodiment, refer to related content of obtaining the weight loss duration in the embodiment shown in FIG. 3. Details are not described herein again.

For example, a workout capability of the user may be represented by a score. Therefore, obtaining the first workout capability of the first user may also be understood as determining a workout capability score of the user. The workout capability score may be referred to as a first workout capability score.

For example, obtaining the first workout capability score of the user may include: obtaining the first workout capability score input by the user.

For example, obtaining the first workout capability score input by a user may include: obtaining the first workout capability score input by the user through a control in a user interface.

For example, the user interface may include a text input control, and the user may input the first workout capability score in the text input control.

For example, the user interface may include a selection control, and the selection control may provide the user with a plurality of workout capability scoring options. The user may select a workout capability score through the selection control. The workout capability score selected by the user may be used as the first workout capability score input by the user.

For example, obtaining the first workout capability score of the user may include: obtaining workout scores of the user of workout items of a plurality of workout types and/or workout scores of each of at least one body part, and determining the first workout capability score based on the workout scores.

For example, the workout scores of the workout items of the plurality of workout types may include an aerobic workout capability score and a strength workout capability score.

For example, an upper limb workout capability score of the user is w, and a weight of the upper limb workout capability score is a; a lower limb workout capability score is x, and a weight of the lower limb workout capability score is b; a core workout capability score is y, and a weight of the core workout capability score is c; and an aerobic workout capability score is z, and a weight of the aerobic workout capability score is d. In this case, the first workout capability score may satisfy the following relation: $s=a*w+b*x+c*y+d*z$, where s represents the first workout capability score, and a, b, c, and d may be preset parameter values.

In an example, obtaining the workout capability score of the workout item of each workout type may include: obtaining the workout capability score of the workout item of each workout type input by the user.

For an implementation of obtaining the workout capability score of the workout item of each workout type input by the user, refer to related content of obtaining the first workout capability score in the foregoing content. Details are not described herein again.

For example, obtaining the workout capability score of the workout item of each workout type may include: obtaining at least one piece of the following information of the user: a quantity of times of performing the workout item within specific duration, obtaining duration required by the user to complete the workout item, or obtaining a body feature parameter value of the user within the duration, for example, a heart rate, a blood pressure, a blood sample, or a maximum oxygen uptake; and calculating the workout capability score based on the at least one type of information.

For example, the quantity of times of performing the workout item within specific duration, the duration required by the user to complete the workout item, or one or more of the body feature parameter values of the user within the duration may be values directly input by the user, for example, values input by the user based on a historical record, or may be obtained by the electronic device by measuring the user in real time.

S620: Determine a first target workout plan based on the first basal metabolism condition, the first fat loss duration, and the first workout capability, where the first target workout plan includes a plurality of target phases, a plurality of pieces of target duration, a plurality of target workout item sets, and a plurality of workout duration ratios, the plurality of pieces of target duration are in a one-to-one correspondence with the plurality of target phases, each piece of target duration in the plurality of pieces of target duration is duration included in the first fat loss duration for a corresponding target phase, the plurality of target workout item sets are in a one-to-one correspondence with the plurality of target phases, each target workout item set in the plurality of target workout item sets includes a workout item in the corresponding target phase, the plurality of workout duration ratios are in a one-to-one correspondence with the plurality of target workout item sets, and each workout duration ratio in the plurality of workout duration ratios is a workout duration ratio between workout items in a corresponding target workout item set.

For example, the plurality of target phases included in the first target workout plan may include at least one of the following phases: a fat loss adaptation period, a metabolism improvement period, a high-efficiency fat-burning period, and an achievement consolidation period. It may be understood that at least one of the following content of these phases is different: duration, a workout type of a workout item in a phase, different workout items of a same workout type, different pieces of workout duration of a same workout type, or different pieces of workout duration of a same workout item.

For example, the fat loss adaptation period is to gradually adapt a body to a workout fat loss rhythm, and intensity is low. The metabolism improvement period is mainly strength training, which is mainly used to improve metabolism; while there might not be significant changes in weight, the body becomes firm and slimmer in shape. The high-efficiency fat-burning period is mainly aerobic training and high-intensity interval training (high-intensity interval training, HIIT), with increased training frequency leading to greater overall calorie consumption and rapid fat loss. The achievement consolidation period is a period in which aerobic training and strength training are balanced, which keeps a muscle mass stable and retains metabolism, reducing a body fat percentage and consolidating the result of a rapid fat-loss period.

For example, determining the first target workout plan based on the first basal metabolism condition, the first fat loss duration, and the first workout capability may include: determining the plurality of target phases, the plurality of pieces of target duration, a plurality of target workout type sets, and the plurality of workout duration ratios based on the first basal metabolism condition and the first fat loss duration, where the plurality of target workout type sets are in a one-to-one correspondence with the plurality of target phases, and each target workout type set in the plurality of target workout type sets includes a workout type in the corresponding target phase; and determining a target workout item set of the corresponding target phase based on the first workout capability and each target workout type set.

For example, determining the plurality of target phases, the plurality of pieces of target duration, the plurality of target workout type sets, and the plurality of workout duration ratios based on the first basal metabolism condition and the first fat loss duration includes: determining the plurality of target phases and the plurality of pieces of target duration based on the first basal metabolism condition and the first fat loss duration; determining a target workout type set of each target phase in the plurality of target phases, to obtain a plurality of target workout type sets that are in a one-to-one correspondence with the plurality of target phases; and determining a workout duration ratio of a plurality of workout types in each target workout type set in the plurality of target workout type sets, to obtain a plurality of target duration ratios that are in a one-to-one correspondence with the plurality of target workout type sets.

For example, the plurality of target phases may be specifically divided based on the first fat loss duration and the first basal metabolism condition.

For example, if the first fat loss duration planned by the user is X weeks and the first basal metabolism condition is low metabolism, the first fat loss duration may be divided into: a fat loss adaptation period, A weeks; a metabolism improvement period, B weeks; a high-efficiency fat-burning period, C weeks; and an achievement consolidation period, D weeks.

For another example, if the first fat loss duration planned by the user is Y weeks and the first basal metabolism condition is high metabolism, the first fat loss duration may be divided into: a fat loss adaptation period, A weeks; a high-efficiency fat-burning period, E weeks; and an achievement consolidation period, F weeks.

It may be understood that the plurality of target phases in the first target workout plan correspond to a plurality of pieces of target duration, and a sum of the plurality of pieces of target duration is the first fat loss duration.

For example, the workout type corresponding to each target phase is determined based on the first workout capability and the first basal metabolism condition.

For example, each of the plurality of target phases corresponds to the corresponding target workout item set. Table 1 shows target workout item sets corresponding to the fat loss adaptation period, the metabolism improvement period, the high-efficiency fat-burning period, and the achievement consolidation period.

Table 1

| Target phase | Target workout item |
|---|---|
| Fat loss adaptation period | Aerobic and strength endurance |
| High-efficiency fat-burning period | Aerobic, strength endurance, and HIIT |
| Metabolism improvement period | Aerobic, strength endurance, and HIIT |
| Achievement consolidation period | Aerobic, strength endurance, and HIIT |

For example, workout duration of each target workout type in the target workout item corresponding to each target phase in the plurality of target phases may be determined based on a weekly training frequency.

It may be understood that each target workout item set includes a plurality of workout types, for example, aerobic, strength endurance, and HIIT. Each workout type corresponds to different pieces of workout duration. The workout duration is determined based on the weekly training frequency.

For example, if the user is in the metabolism improvement period, and the weekly training frequency is K, M aerobic training courses, N strength training courses, and P HIIT training courses are arranged in this phase.

For example, determining the target workout item set corresponding to the target phase based on the first workout capability and each target workout type set may include: determining a first workout difficulty based on a mapping relationship between a first workout capability score and a workout difficulty, where the first workout difficulty is a workout difficulty associated with the first workout capability score; and determining, based on each target workout type set and the first workout difficulty, a target workout item set of a target phase corresponding to each target workout type set, where the target workout item set of the target phase corresponding to each target workout type set includes a workout item that is of each workout type in each target workout type set and whose workout difficulty is the first workout difficulty.

For example, the first workout difficulty includes a strength course difficulty and an aerobic course difficulty, where the strength course difficulty and the aerobic course difficulty may be determined by a user strength score, an aerobic score, a user gender, and a weight. Table 2 and Table 3 are respectively strength course difficulties corresponding to the strength score and the aerobic score.

Table 2

| Strength score | Strength course difficulty |
|---|---|
| [0, a) | X1 |
| [a, b) | Y1 |
| ≥b | Z1 |

Table 3

| Aerobic score | Aerobic course difficulty |
|---|---|
| [0, d) | X2 |
| [d, e) | Y2 |
| ≥e | Z2 |

For example, if the user strength test score is a, and the course difficulty corresponding to the workout capability score is Y1, the strength training with the difficulty Y1 is randomly selected based on the foregoing course type and a course proportion, and arranged in the target workout type. The same applies to aerobic training.

In this embodiment, it may be understood that if the electronic device is a terminal device such as a mobile phone, a tablet, or a wearable device, the step of obtaining various information input by the user may be implemented by the electronic device, or may be received from another terminal device that establishes a communication connection to the electronic device.

In this embodiment, it may be understood that, if the electronic device is a cloud server, a step of obtaining the various information input by the user may be implemented by using a terminal device, and the terminal device receives the information.

In this embodiment, after the first target workout plan is obtained, the first target workout plan may be displayed to the user.

For example, if the electronic device is the mobile phone, the tablet computer, or the like, the first target workout plan may be displayed to the user through a user interface of the electronic device.

Optionally, in a scenario in which the electronic device displays the first target workout plan to the user, the electronic device may further send the first target workout plan to the wearable device, and display the first target workout plan to the wearable device user.

For another example, if the electronic device is a cloud server, the electronic device may send the first target workout plan to various terminal devices such as the mobile phone, the tablet computer, and the wearable device, and display the first target workout plan to the user through the user interface of the electronic device.

For example, the method in this embodiment may further include: obtaining a completion degree of the first target workout plan and/or feedback of the user on the first target workout plan, where the completion degree of the first target workout plan and/or the feedback of the user on the first target workout plan determine a second target workout plan.

In the steps of this embodiment, the second target workout plan may be a plan that is adjusted after the first target workout plan is completed and that is based on a training completion degree and/or the feedback of the user on the first target workout plan.

For example, the completion degree of the first target workout plan may be calculated based on an actual workout condition and a planned condition after training of the plurality of target phases ends

For example, the feedback of the user on the first target workout plan may be a subjective perception level of the user after each time of training is completed.

For example, the perception level may be classified into four levels: easy, just right, strenuous, and challenging.

For example, the feedback of the first target workout plan may be obtained on the user interface.

For example, obtaining the completion degree of the first target workout plan includes: obtaining actual completion duration of each workout item in each of the plurality of target workout item sets; determining planned duration of each workout item in each target workout item set based on the plurality of pieces of target duration and the plurality of workout duration ratios; determining, based on actual completion duration and planned duration of workout items of a same type in each target workout item set, completion degrees of the workout items of the same type in each target workout item set; and determining, based on completion degrees of the workout items of the same type in each target workout item set, a workout completion degree of the target phase corresponding to each target workout item set.

For example, the actual workout situation may be determined by collecting statistics on an actual number of completed groups and an actual number of completed times of different workout types. The planned condition is determined by using a planned number of groups and a planned number of times. The completion degree of the first target workout plan may be calculated by using a weighted value of completion degrees of different types of workout.

For example, a value obtained by multiplying a ratio of an actual number of strength training groups to a planned number of strength training groups by a ratio of an actual number of times to the planned number of times is a completion degree of strength training in the first target workout plan. A value obtained by multiplying a ratio of an actual number of aerobic training groups to a planned number of aerobic training groups by a ratio of an actual number of times to a planned number of times is a completion degree of aerobic training in the first target workout plan. A value obtained by multiplying a ratio of an actual number of HIIT training groups to a planned number of HIIT training groups by a ratio of an actual number of times to a planned number of times is a completion degree of HIIT training in the first target workout plan.

It may be understood that, in this embodiment, the following steps may be repeatedly performed: obtaining a completion degree and/or subjective feedback of the second target workout plan, and adjusting the second target workout plan based on the information, to obtain a new second target workout plan.

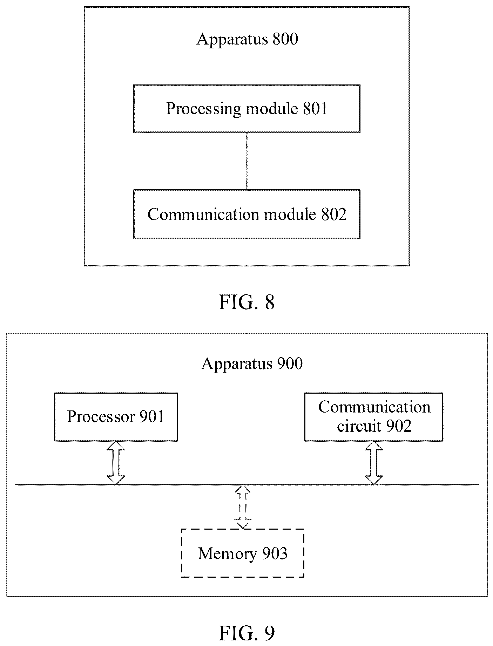

FIG. 8 is a diagram of a structure of an apparatus according to an embodiment of this application. As shown in FIG. 8, the apparatus 800 may include a processing module 801 and a communication module 802.

In a first example, the apparatus 800 may be configured to implement the method implemented in any one of the embodiments shown in FIG. 3 to FIG. 7. For example, the processing module 801 is configured to implement related steps of processing actions in the method described in any one of the embodiments shown in FIG. 3 to FIG. 7, and the communication module 802 is configured to implement related steps of information obtaining actions in the method described in any one of the embodiments shown in FIG. 3 to FIG. 7.

FIG. 9 is a diagram of a structure of an apparatus according to another embodiment of this application. As shown in FIG. 9, the apparatus 900 includes a processor 901 and a communication circuit 902. The processor 901 and the communication circuit 902 are coupled to each other. It may be understood that the communication circuit 902 may be a transceiver or an input/output interface. Optionally, the apparatus 900 may further include a memory 903, configured to store instructions executed by the processor 901, or store input data required by the processor 901 to run the instructions, or store data generated after the processor 901 runs the instructions. It may be understood that the memory 903 may be located outside the processor 901, or may be located inside the processor 901.

In an example, the processor 901 is configured to implement a function of the processing module 801, and the communication circuit 902 is configured to implement a function of the communication module 802.

The apparatus 900 may be an electronic device, or may be an apparatus, a module, a circuit, a chip, or the like disposed in an electronic device, or may be an apparatus that can be used together with an electronic device.

An embodiment of this application provides a computer program product. When the computer program product runs on an electronic device, the electronic device is enabled to perform the technical solutions in the foregoing embodiments. An implementation principle and technical effect of the computer program product are similar to those in the foregoing method-related embodiments. Details are not described herein again.

An embodiment of this application provides a readable storage medium. The readable storage medium includes instructions. When the instructions are run on an electronic device, the electronic device is enabled to perform the technical solutions in the foregoing embodiments. An implementation principle and technical effect of the readable storage medium are similar. Details are not described herein again.

An embodiment of this application provides a chip. The chip is configured to execute instructions. When the chip runs, the technical solutions in the foregoing embodiments are performed. An implementation principle and technical effect of the readable storage medium are similar. Details are not described herein again.

A person of ordinary skill in the art may be aware that, in combination with the examples described in embodiments disclosed in this specification, units and algorithm steps may be implemented by electronic hardware or a combination of computer software and electronic hardware. Whether the functions are performed by hardware or software depends on particular applications and design constraint conditions of the technical solutions. A person skilled in the art may use different methods to implement the described functions for each particular application, but it should not be considered that the implementation goes beyond the scope of embodiments of this application.

It may be clearly understood by a person skilled in the art that, for the purpose of convenient and brief description, for a detailed working process of the foregoing system, apparatus, and unit, refer to a corresponding procedure in the foregoing method embodiments. Details are not described herein again.

In the several embodiments provided in this application, it should be understood that the disclosed system, apparatus, and method may be implemented in other manners. For example, the described apparatus embodiments are merely examples. For example, division into the units is merely logical function division. There may be another division manner during actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electronic, mechanical, or other forms.

The units described as separate parts may or may not be physically separate, and parts displayed as units may or may not be physical units, may be located in one position, or may be distributed on a plurality of network units. Some or all of the units may be selected based on actual requirements to achieve the objectives of the solutions of embodiments.

In addition, the functional units in embodiments of this application may be integrated into one processing unit, or may exist physically separately, or two or more units may be integrated into one unit.

When the functions are implemented in the form of a software functional unit and sold or used as an independent product, the functions may be stored in a computer-readable storage medium. Based on such an understanding, the technical solutions of embodiments of this application essentially, or the part contributing to the conventional technology, or a part of the technical solutions may be implemented in a form of a software product. The computer software product is stored in a storage medium, and includes several instructions for instructing a computer device (which may be a personal computer, a server, a network device, or the like) to perform all or some of the steps of the methods described in embodiments of this application. The foregoing storage medium includes any medium that can store program code, such as a USB flash drive, a removable hard disk, a read-only memory (read-only memory, ROM), a random access memory (random access memory, RAM), a magnetic disk, or an optical disc.

The foregoing descriptions are merely specific implementations of embodiments of this application. However, the protection scope of embodiments of this application is not limited thereto. Any change or replacement readily figured out by a person skilled in the art within the technical scope disclosed in embodiments of this application shall fall within the protection scope of embodiments of this application. Therefore, the protection scope of embodiments of this application shall be subject to the protection scope of the claims.

**Claims**

1. A health plan formulation method, wherein the method is applied to an electronic device and comprises:

   obtaining a target weight loss rate proportion of a user and a current weight of the user, wherein the target weight loss rate proportion is a weight loss rate proportion expected by the user, and the weight loss rate proportion is a proportion of a weight loss in first unit duration in the current weight;
   determining weight loss fat content corresponding to the target weight loss rate proportion, wherein the weight loss fat content is a proportion of a fat amount in a weight loss in the weight loss; and
   determining a calorie deficit of the user in second unit duration based on the weight loss fat content corresponding to the target weight loss rate proportion, the target weight loss rate proportion, the current weight of the user, fat calories per unit weight, and lean body calories per unit weight.

2. The method according to claim 1, wherein obtaining the target weight loss rate proportion of the user comprises:

   obtaining a target weight loss rate of the user and the current weight of the user, wherein the target weight loss rate is a weight loss rate expected by the user, and the weight loss rate is a weight loss in the first unit duration; and
   determining the target weight loss rate proportion based on the target weight loss rate of the user and the current weight of the user.

3. The method according to claim 1 or 2, wherein determining the weight loss fat content corresponding to the target weight loss rate proportion comprises:

   determining a group category to which the user belongs;
   determining a first mapping relationship from a plurality of mapping relationships based on the group category to which the user belongs, wherein the first mapping relationship is a mapping relationship corresponding to the group category to which the user belongs in the plurality of mapping relationships, the plurality of mapping relationships are in a one-to-one correspondence with a plurality of group categories, and each mapping relationship in the plurality of mapping relationships indicates a mapping relationship between a fat proportion in a weight loss of a user of a corresponding group category and a weight loss proportion of the user of the corresponding group category in the first unit duration; and
   determining, based on the first mapping relationship, the weight loss fat content corresponding to the target weight loss rate proportion.

4. The method according to claim 3, wherein determining the group category to which the user belongs comprises:

   obtaining user information, wherein the user information comprises at least one piece of the following information of the user: a maximum oxygen uptake, a body fat percentage, an age, a gender, the current weight, and a body mass index; and
   determining, based on the user information, the group category to which the user belongs.

5. The method according to any one of claims 1 to 4, wherein the weight loss fat content corresponding to the target weight loss rate proportion, the target weight loss rate proportion, the current weight of the user, the fat calories per unit weight, the lean body calories per unit weight, and the calorie deficit of the user in the second unit duration satisfy the following relation:

$$g=(w*v*p*k1+w*v*(1-p)*k2)/t,$$

   wherein
   g represents the calorie deficit, w represents the current weight of the user, v represents the target weight loss rate proportion, p represents the weight loss fat content corresponding to the target weight loss rate proportion, k1 represents the fat calories per unit weight, k2 represents the lean body calories per unit weight, and t represents a ratio of the first unit duration to the second unit duration.

6. The method according to any one of claims 1 to 5, wherein the method further comprises:
   determining a diet plan and a workout plan of the user in the second unit duration based on the calorie deficit and basal metabolism calories of the user in the second unit duration.

7. A health plan formulation method, applied to an electronic device or a cloud server, wherein the method comprises:

obtaining a first basal metabolism condition, first fat loss duration, and a first workout capability, wherein the first basal metabolism condition is a basal metabolism condition of a user for whom a health plan is to be formulated, the first workout capability is a workout capability of the user for whom the health plan is to be formulated, and the first fat loss duration is fat loss duration expected by the user for whom the health plan is to be formulated; and determining a first target workout plan based on the first basal metabolism condition, the first fat loss duration, and the first workout capability, wherein the first target workout plan comprises a plurality of target phases, a plurality of pieces of target duration, a plurality of target workout item sets, and a plurality of workout duration ratios, the plurality of pieces of target duration are in a one-to-one correspondence with the plurality of target phases, each piece of target duration in the plurality of pieces of target duration is duration comprised in the first fat loss duration for a corresponding target phase, the plurality of target workout item sets are in a one-to-one correspondence with the plurality of target phases, each target workout item set in the plurality of target workout item sets comprises a workout item in the corresponding target phase, the plurality of workout duration ratios are in a one-to-one correspondence with the plurality of target workout item sets, and each workout duration ratio in the plurality of workout duration ratios is a workout duration ratio between workout items in a corresponding target workout item set.

8. The method according to claim 7, wherein determining the first target workout plan based on the first basal metabolism condition, the first fat loss duration, and the first workout capability comprises:

determining the plurality of target phases, the plurality of pieces of target duration, a plurality of target workout type sets, and the plurality of workout duration ratios based on the first basal metabolism condition and the first fat loss duration, wherein the plurality of target workout type sets are in a one-to-one correspondence with the plurality of target phases, and each target workout type set in the plurality of target workout type sets comprises a workout type in the corresponding target phase; and determining a target workout item set of the corresponding target phase based on the first workout capability and each target workout type set.

9. The method according to claim 8, wherein determining the plurality of target phases, the plurality of pieces of target duration, the plurality of target workout type sets, and the plurality of workout duration ratios based on the first basal metabolism condition and the first fat loss duration comprises:

determining the plurality of target phases and the plurality of pieces of target duration based on the first basal metabolism condition and the first fat loss duration; determining a target workout type set of each target phase in the plurality of target phases, to obtain the plurality of target workout type sets that are in a one-to-one correspondence with the plurality of target phases; and determining a workout duration ratio of a plurality of workout types in each target workout type set in the plurality of target workout type sets, to obtain a plurality of target duration ratios that are in a one-to-one correspondence with the plurality of target workout type sets.

10. The method according to claim 8 or 9, wherein determining the target workout item set of the corresponding target phase based on the first workout capability and each target workout type set comprises:

obtaining a first score based on the first workout capability, wherein the first score is a score of the first workout capability; determining a first workout difficulty based on a mapping relationship between a workout capability score and a workout difficulty, wherein the first workout difficulty is a workout difficulty associated with the first score; and determining, based on each target workout type set and the first workout difficulty, a target workout item set of a target phase corresponding to each target workout type set, wherein the target workout item set of the target phase corresponding to each target workout type set comprises a workout item that is of each workout type in each target workout type set and whose workout difficulty is the first workout difficulty.

11. The method according to any one of claims 9 and 10, wherein the method further comprises: obtaining a completion degree of the first target workout plan and/or feedback of the user on the first target workout plan, and determining a second target workout plan.

12. The method according to claim 11, wherein obtaining the completion degree of the first target workout plan comprises:

obtaining actual completion duration of each workout item in each of the plurality of target workout item sets;

determining planned duration of each workout item in each target workout item set based on the plurality of pieces of target duration and the plurality of workout duration ratios;

determining, based on actual completion duration and planned duration of workout items of a same type in each target workout item set, completion degrees of the workout items of the same type in each target workout item set; and

determining, based on the completion degrees of the workout items of the same type in each target workout item set, a workout completion degree of the target phase corresponding to each target workout item set.

13. An electronic device, comprising:

one or more processors; and
one or more memories, wherein
the one or more memories store one or more computer programs, the one or more computer programs comprise instructions, and when the instructions are executed by the one or more processors, the electronic device is enabled to perform the method according to any one of claims 1 to 12.

14. A computer-readable storage medium, wherein the computer-readable storage medium comprises a computer program or instructions, and when the computer program or the instructions are run on a computer, the method according to any one of claims 1 to 12 is performed.

15. A computer program product, wherein the computer program product comprises a computer program or instructions, and when the computer program or the instructions are run on a computer, the method according to any one of claims 1 to 12 is performed.

Electronic device 100

Antenna 1

Antenna 2

| Mobile communication module 2G/3G/4G/5G [150] | | Wireless communication module BT/WLAN/GNSS/NFC/IR/FM [160] |

| Speaker [170A] | | | |
| Receiver [170B] | Audio module [170] | Processor [110] | Sensor module [180] |
| Microphone [170C] | | | Pressure sensor [180A] |
| Headset jack [170D] | | | Gyroscope sensor [180B] |
| Displays 1 to N [194] | | | Barometric pressure sensor [180C] |
| Cameras 1 to N [193] | | | Magnetic sensor [180D] |
| Indicator [192] | | | Acceleration sensor [180E] |
| Motor [191] | | | Distance sensor [180F] |
| Button [190] | | | Optical proximity sensor [180G] |
| Internal memory [121] | | | Fingerprint sensor [180H] |
| SIM card interfaces 1 to N [195] | | | Temperature sensor [180J] |
| Interface [120] for external memory | | | Touch sensor [180K] |

| USB interface [130] | Charging management module [140] | Power management module [141] | Ambient optical sensor [180L] |
| Charging input | | Battery [142] | Bone conduction sensor [180M] |

FIG. 1

FIG. 2

Obtain a target weight loss rate proportion of a user and a current weight of the user, where the target weight loss rate proportion is a weight loss rate proportion expected by the user, and the weight loss rate proportion is a proportion of a weight loss in first unit duration in the current weight ⌐∿ S310

Determine a weight loss fat content corresponding to the target weight loss rate proportion, where the weight loss fat content is a proportion of a fat amount in a weight loss in the weight loss ⌐∿ S320

Determine a calorie deficit of the user in second unit duration based on the weight loss fat content corresponding to the target weight loss rate proportion, the target weight loss rate proportion, the current weight of the user, fat calories per unit weight, and lean body calories per unit weight ⌐∿ S330

FIG. 3

Body fat percentage:
• a% to b%
• c% to d%
• ...

Body mass index:
• e to f
• g to h
• ...

Age:
• i to j
• k to l
• ...

Gender:
• Male
• Female

...

Obese group
Body fat percentage: a% to b%, body mass index: e to f, gender: male

Weight loss fat proportion

Weight loss rate proportion

Healthy group
Body fat percentage: c% to d%, body mass index: g to h, gender: male

Weight loss fat proportion

Weight loss rate proportion

...

FIG. 4

EP 4 745 979 A1

**Your height**

164 cm

165 cm

166 cm

OK

**Your weight**

64　0 kg

65　1 kg

66　2 kg

OK

**Expected weight loss rate**

0.3 to 0.6 kg/week
(recommended)

0.7 to 1.0 kg/week (fast)
Weight proportion: 1.1 to 1.5%
A fat loss proportion is medium,
which requires great perseverance
and takes a maximum period of
12 weeks

1.1 to 1.3 kg/week (radical)

OK

**Target weight**

Estimated to
complete in 7
weeks

59　0 kg

60　1 kg

61　2 kg

OK

**Weight loss target**

- Daily calorie deficit　　550 kcal
- Weight loss per week　0.7 to 1.0 kg
- Duration　　6 weeks
- Target weight　　60 kg

**Diet Plan**

- Daily intake　　1350 kcal
- Breakfast　　405 kcal
- Lunch　　540 kcal
- Dinner　　338 kcal
- Snack　　67 kcal

**Workout Plan**
- Consumption of a
  recommended activity　　550 kcal

70% of aerobic workout is
recommended, such as walking,
running, and cycling
30% of strength training is
recommended, such as core
strengthening and upper limb
strength training

**FIG. 5**

Obtain a first basal metabolism condition, first fat loss duration, and a first workout capability, where the first basal metabolism condition is a basal metabolism condition of a user for whom a health plan is to be formulated, the first workout capability is a workout capability of the user for whom the health plan is to be formulated, and the first fat loss duration is fat loss duration expected by the user for whom the health plan is to be formulated

~ S610

Determine a first target workout plan based on the first basal metabolism condition, the first fat loss duration, and the first workout capability, where the first target workout plan includes a plurality of target phases, a plurality of pieces of target duration, a plurality of target workout item sets, and a plurality of workout duration ratios, the plurality of pieces of target duration are in a one-to-one correspondence with the plurality of target phases, each piece of target duration in the plurality of pieces of target duration is duration included in the first fat loss duration for a corresponding target phase, the plurality of target workout item sets are in a one-to-one correspondence with the plurality of target phases, each target workout item set in the plurality of target workout item sets includes a workout item in the corresponding target phase, the plurality of workout duration ratios are in a one-to-one correspondence with the plurality of target workout item sets, and each workout duration ratio in the plurality of workout duration ratios is a workout duration ratio between workout items in a corresponding target workout item set

~ S620

FIG. 6

S701: Obtain a BMI, a body fat percentage, a gender, and an age

↓

S702: Determine a normal body fat percentage range

↓

S703. Determine whether the body fat percentage of a user is higher than a maximum value of the normal body fat percentage range

Yes →

S704: Determine that a first basal metabolism condition is high metabolism

No ↓

S705: Determine whether the body fat percentage of the user is lower than a minimum value of the normal body fat percentage range

Yes →

S706: Determine that a first basal metabolism condition is low metabolism

No →

S707: Determine that a first basal metabolism condition is standard metabolism

FIG. 7

Apparatus 800

Processing module 801

Communication module 802

FIG. 8

Apparatus 900

Processor 901

Communication circuit 902

Memory 903

FIG. 9

## INTERNATIONAL SEARCH REPORT

| | International application No. |
| --- | --- |
| | **PCT/CN2024/108534** |

### A. CLASSIFICATION OF SUBJECT MATTER

G16H 20/30(2018.01)i

According to International Patent Classification (IPC) or to both national classification and IPC

### B. FIELDS SEARCHED

Minimum documentation searched (classification system followed by classification symbols)

IPC:G16H

Documentation searched other than minimum documentation to the extent that such documents are included in the fields searched

Electronic data base consulted during the international search (name of data base and, where practicable, search terms used)

VEN, CNABS, CNTXT, WOTXT, USTXT, CNKI, IEEE: 方案, 计划, 目标减重, 速率占比, 当前体重, 脂肪含量, 热量, 瘦体, 映射, 代谢, protocol, schedule, target weight, rate fraction, current body weight, fat content, calorie, lean body, mapping, metabolism

### C. DOCUMENTS CONSIDERED TO BE RELEVANT

| Category* | Citation of document, with indication, where appropriate, of the relevant passages | Relevant to claim No. |
| --- | --- | --- |
| X | CN 112365955 A (BEIJING CALORIE TECHNOLOGY CO., LTD.) 12 February 2021 (2021-02-12) <br> description, paragraphs [0031]-[0070] and [0090]-[0096], and figures 1-2 | 1-6, 13-15 |
| A | CN 112735561 A (SHENZHEN LUOHU HOSPITAL GROUP et al.) 30 April 2021 (2021-04-30) <br> entire document | 1-15 |
| A | CN 109754865 A (TONGFANG HEALTH TECHNLOGY (BEIJING) CO., LTD.) 14 May 2019 (2019-05-14) <br> entire document | 1-15 |
| A | CN 113254774 A (SHENZHEN XIAOLAI INTELLIGENT TECHNOLOGY CO., LTD.) 13 August 2021 (2021-08-13) <br> entire document | 1-15 |
| A | CN 110706784 A (ANHUI HUAMI INFORMATION TECHNOLOGY CO., LTD.) 17 January 2020 (2020-01-17) <br> entire document | 1-15 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **10 September 2024** | **14 September 2024** |

| Name and mailing address of the ISA/CN | Authorized officer |
| --- | --- |
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/108534** |

**C.    DOCUMENTS CONSIDERED TO BE RELEVANT**

| Category* | Citation of document, with indication, where appropriate, of the relevant passages | Relevant to claim No. |
| --- | --- | --- |
| A | US 2016074707 A1 (CAMBIA HEALTH SOLUTIONS, INC.) 17 March 2016 (2016-03-17) entire document | 1-15 |

Form PCT/ISA/210 (second sheet) (July 2022)

**INTERNATIONAL SEARCH REPORT**
Information on patent family members

International application No.

**PCT/CN2024/108534**

| Patent document cited in search report | | | Publication date (day/month/year) | Patent family member(s) | Publication date (day/month/year) |
|---|---|---|---|---|---|
| CN | 112365955 | A | 12 February 2021 | None | |
| CN | 112735561 | A | 30 April 2021 | None | |
| CN | 109754865 | A | 14 May 2019 | None | |
| CN | 113254774 | A | 13 August 2021 | None | |
| CN | 110706784 | A | 17 January 2020 | None | |
| US | 2016074707 | A1 | 17 March 2016 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311168990